# EUROPEAN PATENT APPLICATION

(11) **EP 1 314 448 A1**
(43) Date of publication of application: **28.05.2003**
(21) Application number: 01981026.6
(22) Date of filing: 08.11.2001
(51) Int. Cl.: A61N 1/04, A61N 1/32

(54) **PULSE HEALTH INSTRUMENT**

(30) Priority: 28.05.2001 JP 2001159179
(71) Applicant: YA-MAN LTD, Koto-ku, Tokyo 135-0045 (JP)
(72) Inventor: YAMAZAKI, Iwao, YA-MAN LTD., Koto-ku, Tokyo 135-0045 (JP); YAMAZAKI, Akitsugu, YA-MAN LTD., Koto-ku, Tokyo 135-0045 (JP); YAMAZAKI, Kimiyo, YA-MAN LTD., Koto-ku, Tokyo 135-0045 (JP)
(74) Representative: Granleese, Rhian Jane
(86) International application number: JP0109776
(87) International publication number: WO02096510

(57) **Abstract**

A pulse health device which can impart a sufficient treatment efffect even to the periphery of waist part where waist part, hip part and abdoment part having an irregular shape exist closely to each other. An electrode belt 5 of the pulse health device 1 has has a slit 57 made along the longitudinal direction thereof between planar electrodes H1, H3 on the uper stage side and planar electrodes H2, H4 on the lower stage side. When the electrode belt 5 is applied to the periphery of waist part where the parts of a human body having an irregular shape exist closely to each other, the pulse health device 1 can absorb variation of shape through the slit 57 and since the planar electrodes H1, H2, H3 and H4 can touch the peripehry of the waist part stably, a sufficient treatment effect can be imparted.

## Description

### Field of the Invention

The present invention relates to a pulse health device for improving health of the user by pulse treatment, and particularly to a pulse health device for cosmetic treatment of a body of human beings by applying electrically pulse current to the body of the human beings.

### Description of the Prior Art

A biologícal electric current flows always so as to maintain the cells active and the muscle to contract and relax. The pulse health device supplies artificially a pulse current into the living body from the outside so as to stimulate the living body, thereby activating the cells as the natural biological current does, to make thereby the muscles contract and relax to enhance the activity of the living cells and to stimulate the body of human beings.

This kind of a pulse health device has been developed along various types to adjust various application figures. For example, there has been proposed a belt-installation type with electrodes mounted on the internal side of the belt to supply pulse current. This type of the pulse health device can enable to provide a treatment during everyday life and exercising by installing the belt on their body.

However, the above pulse health device with belt installation can provide merely small range of the surface of the body within which the belt can be wrapped on. Moreover, this pulse health device can not provide the electrodes for supply of pulse under stable condition. Therefore, the prior art pulse health device can not give the sufficient treatment on such range having many and much projections.

Further, this kind of a pulse health device needs generally each electrode provided on a belt, a device member for supplying pulse current to the electrodes, and a plurality of cables to connect the body member to each electrode. Therefore, the prior art pulse health device will take much time and care to start the treatment, and further, the user can not move freely nor exercise during the treatment, so that there is much limitation of the application.

Further, the electrodes as mounted on the belt are formed by applying carbon material and the like on the surface of urethane rubber to form a coating as a planar electrode. This kind of a planar electrode has to be fixed for example by sawing the electrode on the belt. Then, the electrode must have some uneven margin or projected edge on its peripheral, so that strong pulse current can be charged on such projected edge, or such uneven margin or the projected edge of the electrode can make the skin of the user contact, that may make bad feeling on the user during its installation.

The present invention has been developed so as to solve the above mentioned problems.

It is an object of the present invention to provide a pulse health device with simplified structure, which enables to treat simultaneously a plurality of positions of the body.

It is another object of the present invention to provide a pulse health device, which enables to give sufficient treatment even to the portions having curved surfaces and/or much projections near each the other, such as waist, buttocks and abdomen.

It is other object of the present invention to provide a pulse health device in which the setting before starting the treatment can be easily attained, and further, the freedom of moving and exercise of the user even when the user is treated.

It is further object of the present invention to provide a pulse health device which improves the contact feeling and fitness to the body.

### Disclosure of Invention

In accordance with the present invention, a pulse health device comprises a pulse generator for generating pulse current a belt provided with a plurality of electrodes for providing the pulse current as generated by the pulse generator to a body of a user, the belt having slits.

In accordance with the present invention, even when the belt with the electrodes has to be applied to a projected position of the body, such as, waist, buttocks and abdomen, the slits can adapt such projections of the body. Therefore, the health device of the present invention can make intimate and stable contact of the pulse supply electrodes to such projections of the body by simple structure of the device, so that sufficient treatment effect can be expected.

In accordance with the present invention, the pulse health device can be installed on the body with the belt covering widely to make the plurality of electrodes contact relatively wide portions of the body. Then, the pulse device can enable to treat relatively wide ranges of the body.

Further, in accordance with the present pulse health device the said slits are provided not reaching to the margin of the belt.

That is, the slits are formed merely the middle of the belt. Therefore, the pulse device can make both ends of the belt connecting so to form one strap with the middle thereof broaden. Then, the belt can be easily installed or contacted widely on the broaden parts of the body, so that the belt can be easily handled, and further the plurality of the electrodes for supply of pulse current can be contacted on the plurality of positions of the body under stable condition.

In accordance with the present health device, said slits can be formed from the margin of the belt toward the inside thereof.

That is, the pulse device enables to enhance the freedom of belt installation on the body so that the electrodes for supply of pulse current can be surely contacted on the target positions of the body to be treated, under stable condition.

In accordance with the present invention, said slits can be formed along the longer direction of the belt.

That is, the inventive pulse health device has the direction in which the belt should be wrapped on the body, rectangular (normal) to the direction in which the belt should be broadened by making the slit wide. Therefore, even when the belt is wrapped by strong tension, the desired position at which the electrode is positioned is not any way shifted from the fixed desired position. Then, the target position at which the electrode should be positioned for example, for the purpose of shaping-up can be corrected established in contact, so that the sufficient effect can be expected.

In accordance with the present pulse health device, said slit can be formed among at least two of the said electrodes as formed on the belt.

The inventive pulse health device can enable to supply pulse current e.g. under different conditions, e.g. the two electrodes which are formed at opposite side of the slit each the other. Therefore, this pulse health device enables to supply each appropriate pulse patterns to each of the electrode contacted each of the body, so that the treatment effect can be enhanced.

In accordance with the present invention, the health device is provided with said pulse generator and output connector or connectors for supply of pulse current, the output connector(s) is mounted detachably on said belt, and further input connector(s) mountablly provided on said belt, which can mount said device through said output connector.

Further, in this health device, said belt is provided with a plurality of the electrodes which connects respectively said input connectors.

That is, the inventive pulse health device does not need any cable connecting the device to the belt, and therefore, the setting for starting the treatment can be easily attained, and further can enhance the freedom of the handling by the device, so that the user can move when treated, and further, can exercise even when treated.

In this health device, the electrodes are formed as conductive sheets with the margin thereof being coated by insulating coatings.

Further, in this health device, at least one part of said conductive sheet (s) is exposed from said insulating coatings so as to form input terminal(s) for said pulse current.

The health device in accordance with the present invention, the insulating coatings are formed as a coating of insulation on the surface of the conductive sheet(s).

Further, in the health device in accordance with the present invention, the insulating coatings are formed by coating insulating paint on the surface of said conductive sheet(s).

Then, in the health device of the present invention the insulating coatings are formed by thermal-melt coating of a meltable material having insulation property, on the surface of said conductive sheet(s).

Further, in the health device of the present invention the insulating coatings are formed by adhering insulating film(s) on the surface of said conductive sheet(s).

In accordance with the present invention, the insulating coatings can be formed by coating insulating photo-sensitive material on the surface of said conductive sheet(s), and exposing through a pattern of electrode(s) to be formed, and developing and fixing.

Further, in accordance with the present invention, the insulating coatings can be formed by adhering insulating photo-sensitive thin film on the surface of said conductive sheet(s), and exposing through a pattern of electrode(s) to be formed, and developing and fixing.

Accordingly, the pulse health device of the present invention can solve the problems of direct contacts to the skin of the user, which problems are occurred by unevenness or projected edge of the planar electrode, resulting from electrode direct adhering to the belt. Then, the contact feeling when the belt is installed can be well improved.

### Brief Description of the Drawings

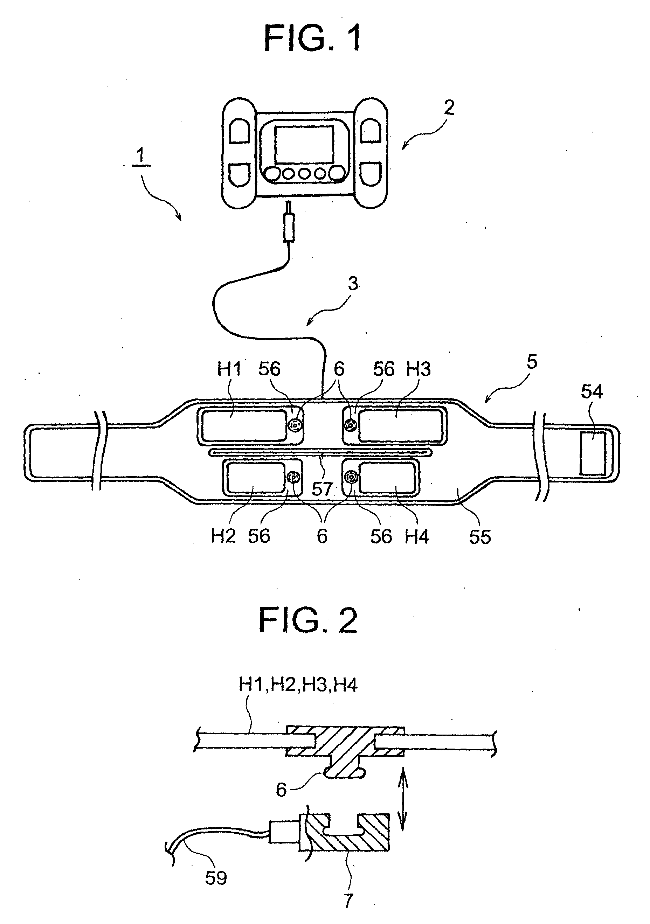

A more detailed description of the invention is facilitated by reference to the drawings which form a part of this specification and wherein: FIG. 1 shows a whole composition of the pulse health device of the first embodiment of the present invention.
FIG. 2 shows a structure of the contact part of the conductive belt provided with the pulse health device of FIG. 1.

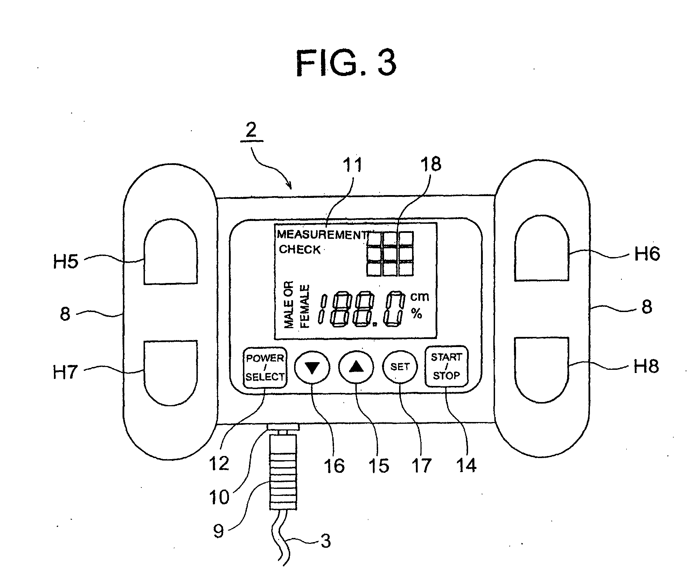

FIG. 3 shows a plane view of a control unit provided with the pulse health device of FIG. 1.
FIG. 4 is a functional block diagram of the pulse health device of FIG. 1.
FIG. 5 shows a circuit diagram of the unit for measuring impedance as provided in the pulse health device.
FIG. 6 shows a circuit block diagram of the unit for treating in the health device of FIG. 1.
FIG. 7 shows use of the health device of FIG. 1
FIG. 8 shows a plane view of an alternative electrode belt different from the device of FIG. 1.
FIG. 9 shows a plane view of another alternative electrode belt different from the device of FIG. 1 and 8.
FIG. 10 shows a front view of a control unit provided with the pulse health device of the second embodiment.
FIG. 11 shows a back view of the control unit of FIG. 10.
FIG. 12 shows an illustrative view where the control unit of FIG. 10 is mounted on the waist position together with the conductive belt.
FIG. 13 shows a part plane view of the conductive belt provided with the pulse health device of the third embodiment.
FIG. 14 shows a partly sectional view of the planar electrode provided on the conductive belt of FIG. 13.

### Detailed Description of Preferred Embodiments

In reference with the attached drawings, the present invention will be detailedly described.

### (First Embodiment)

FIG. 1 shows a schematic view of whole arrangement of a pulse health device of the first embodiment of the present invention.

As shown in the drawings, this pulse health device 1 can treat to shape up the body of the user by applying pulse current to the body. The pulse health device 1 comprises a portable control unit 2, and an electrode belt 2 the electrodes of which are connected to the control unit 2 through a cable 3.

An electrode belt 5 has four planar electrodes H1, H2, H3 and H4. Those planar electrodes H1, H2, H3 and H4 are to contact to the approximation of the waist of the user ( waist, buttocks and abdomen). The planar electrodes H1 and H2 positioned right side upper and lower levels on the belt 5 are for supply of pulse current, and the planar electrodes H3 and H4 positioned left side upper and lower levels on the belt 5 are for detecting electrode.

The planar electrodes H1, H2, H3 and H4 are as shown in FIG. 2 having a button projection 6 . Those button projections 6 correspond to a button recess 7 connecting a cable 59 and cable 3 with detachable structure. Therefore, the planar electrodes H1, H2, H3 and H4 are connected electrically to a control unit 2 through the button projection 6, button recess 7, cables 59 and 3.

The structure of the electrode belt 5 will be explained in the below description.

On the other hand, the control unit 2 has a pair of handles 8 at the both right and left ends thereof. Each handle 8 has a handle electrodes H5 and H6 for supply of pulse, and handle electrodes H7 and H8 for detect of pulse. The control unit 2 has a connector 10 for connecting retractably a connecting jack 9 of a cable 3.

The control unit 2 has further an LCD display 11 between the right and left handles 8. The control unit 2 has further a power/select a button 12 for turning on or off the power, and selecting the function of the device, at the position below the LC display 11, a start/stop button 14 for starting and stopping the measurement of body fatness of the user, and a up key 15, a down key 16 and a set key 17 for input and setting of the personal data of the user, such as female or male, age, length, weight of the user, and further the strength, wave number of pulse current for the treatment, and the period of the treatment.

The display 11 can indicate treatment modes for example "rubbing", "reducing", "developing", "pressing", "knocking", "pressing", "forging or hullering" in the order of weaker to stronger, in graphic display for the user to select the mode of treatment by changing the height of pulse current by a select button (not shown).

Further, the display 11 has an indication of type of "somatoplasm" of the user, such as "muscularity", "ideal healthy type", "emaciation type" and "adiposis type" as judged easily from the visual information of the user.

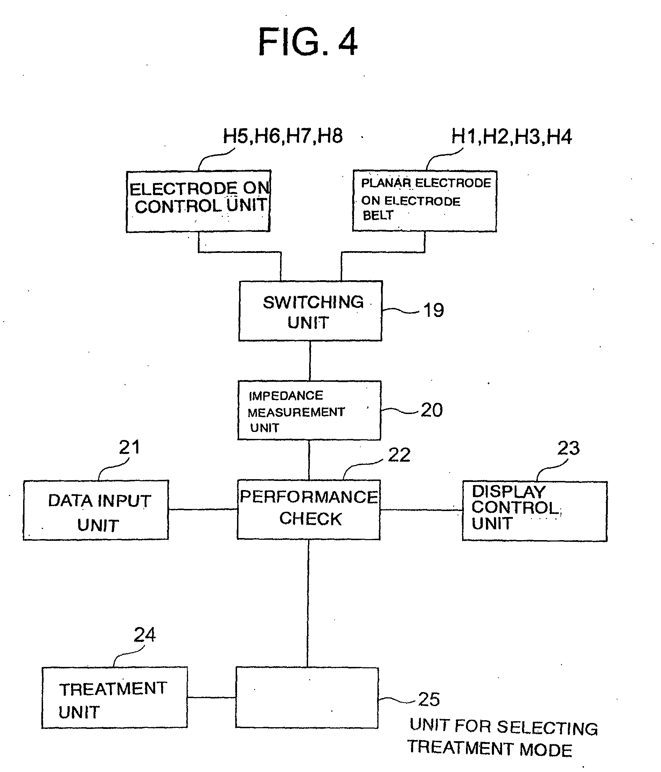

FIG. 4 shows a schematic block diagram of the pulse health device.

The pulse health device 1 has as shown in the drawings, a switching unit 19, an impedance measurement unit 20, a unit for judging "somatoplasm" of the user 22, a display control unit 23, a treatment unit 24, and a unit for selecting the treatment purpose 25.

A swtiching unit 19 will switch the connections between the electrodes of the control unit 2 and the electrodes mounted on the electrode belt 5.

The measurement unit 20 for measuring the impedance of the body of the use is to measure the impedance between the positions as given on the body of the user by applying alternating voltage having 50 kHz sign curve voltage, and detecting the voltage from the detect electrodes.

The unit 22 for judging "somatoplasm" of the user will determine the "somatoplasm" of the user by determining a body fatness of the user and the "somatoplasm" (physique) of the user on the basis of a personal information such as male or female, age, length and weight of the user.

The display control unit 23 will show the "somatoplasm" of the user, fatness of the user, and the other information of the user, on the display 11 of the control unit 2.

The treatment unit 24 will stimulate the body of the user by applying pulse current to the body through the group of the planar electrodes provided by the control unit 2 and on the electrode belt 5.

The unit 25 for selecting the treatment purpose will select an appropriate treatment purpose to be done by the treatment unit 24 in accordance with the "somatoplasm" of the user as determined by the unit 22 for judging "somatoplasm" of the user.

The treatment patterns to be selected by the selection unit 25 can be categorized as "toning" and "drainage" so called.

"Toning" is a treatment by applying a low frequency pulse with 5 to 10 Hz to stimulate the deep portions of the body, thereby, accelerating a blood flow by moving the skeltal muscle of the user, and giving relax to the body.

"Drainage" is a treatment by applying a high frequency pulse with 20 to 100 Hz to stimulate the surface portions of the body, thereby, accelerating a lymph flow by moving the superstructure muscle of the user, and thereby removing the edema of the body.

The "toning" and "drainage" as described above can be further divided depending to the pulse frequency to be applied. The pulse health device of the present invention can select an appropriate treatment purpose by an appropriate frequency of pulse to be applied to the user, on the basis of the information, in accordance with the " somatoplasm " of the user as judged by the judgement unit 22. Therefore, this pulse health device can give a given treatment effect to any kind of the user, or any kind of somatoplasm of the user.

The "toning" and "drainage" can have special "toning" and "drainage" treatment in which the pulse voltage is cyclically raised and reduced so as to change the stimulating level. Further, a sequential or time series "toning" and "drainage" can be applied in which the pulse with frequency being changing sequentially or in time series between the plurality of the electrodes. Further, a time-division "toning" and "drainage" can be applied in which time-division pulse voltage is charged at the same time between the plurality of the electrodes. Therefore, the user can select among such variety of treatment patterns.

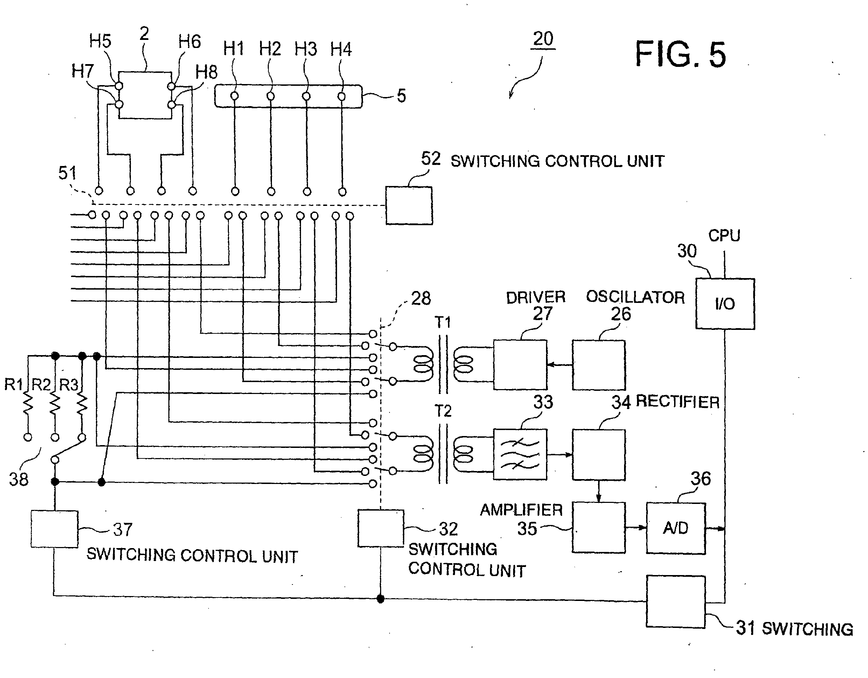

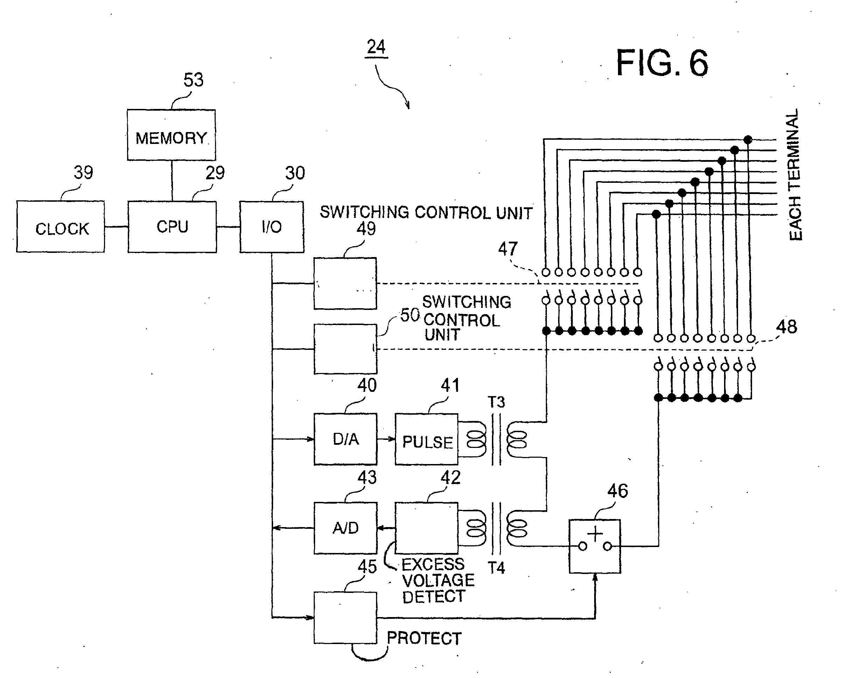

In reference to FIGS. 5 and 6, the impedance measurement unit 20 and the treatment unit 24 will be described.

The measurement of body impedance can be exerted by generating alternating sign wave oscillating voltage output from an oscillator 26 of the impedance measurement unit 20, and applying this oscillating voltage output to the supply electrodes H1, H2, H5 and H6 of the belt 5 and the control unit 2 through a driving circuit 27, transformer T1, and a switching unit 28.

Further, the measurement of body impedance is carried out under such condition that the left and right handles 8 are contacted by the hands of the user, at the same time the user has to touch the supply electrodes H5 and H6, and further the detect electrodes H7 and H8 with his fingers, and further, the belt 5 has to be wrapped around his waist.

When the user pushes the start/stop button 14 under the above mentioned state, the switch 28 will turn the circuit for measurement of the impedance on, charging the detect voltage in the electrodes H3, H4, H7 and H8 from the control unit 2.

The impedance between both hands of the user, the impedance among the positions of the waist, and the impedance between the hand and the waist should be separately measured so that selecting of switch 28 and the switching timing should be controlled.

That is, a switching control signal from CPU 29 of the treatment unit 24 will enter into the switching control circuit 32 through I/O interface 30, a switching unit 31, and then the switching control circuit 32 will change the switch 28 on the basis of the switching control signal.

The alternating current as generated in the electrodes H3, H4, H7 and H8 will be transformed into a continuous current through a switch 28, transformer T2, a band pass filter 33, a rectifier circuit 34, an amplifier 35, and then wave shape regulated, level -adjusted, off-set adjusted, and then transformed into a digital signal with A/D transformer 36, and put into CPU 29, via I/O interface 30. Thereby, the impedance between left and right hands, the impedance among the positions around the waist, and the impedance between the hand and the position around the waist can be measured separately.

Before measurement of the impedance, the output properties of the detect circuit should be corrected so as to reduce the measurement errors due to the elapsed time of the measurement circuit and the temperature properties.

The relationship of the three parameter of body impedance Z (the impedance between left and right hands, the impedance among the positions around the waist, and the impedance between the hand and the position around the waist) with the detected altering voltage V detected by the detect circuit can be referred to the regression line equation z = k • V + C0.

The given altering voltage same as to that in which the body impedance is measured is charged between the both terminals of the circuit of three dummy resistances R1, R2 and R3 as known, and the alternating voltage V as generated between the both terminals of the circuit of the three dummy resistances R1, R2 and R3 is detected to determine the relative constant k of the regression line and the fixed constant C0 .

The CPU 29 will switch a switch 38 through an I/O interface circuit 30, a switching unit 31 and a switching control circuit 37 and then, the connection with the secondary terminals of a transformer T1 and the secondary terminals of a transformer T will switch from the electrodes H3, H4, H7 and H8 for the detect side to the dummy resistances R1, R2 and R3, so as to correct the output properties in the detect circuit. Further, the CPU 29 will change a switch 28 through the switching control circuit 32 into the three dummy resistances R1, R2 and R3.

The CPU 29 will determine a frequency demultiplication of a clock pulse of a standard clock generator 39 corresponding to a treatment purpose as selected by a treatment pattern selector 25 on the basis of program exerted in a memory 63 of the treatment exertion unit 24. The CPU will supply the digital signals demultiplied by the frequency demultiplication to a pulse generator 41 through an I/O interface 30 and D/A converter 40. The pulse generator 41 will generate a frequency and a voltage pulse corresponding to the selected treatment purpose, and supply to a primary side of the transformer T3.

The secondary terminals of the transformer T3 connect serially a primary coil of a transformer T4, and the secondary coil of the transformer T4 connects a current detecting circuit 42 for detecting the excess current. The current as detected by the current detecting circuit 42 will be put in the CPU 29 through an A/D converter 43 and I/D interface 30. The CPU 29 will break down the cutout switch 46 by a current protection circuit 45, when the current value exceeds the standard value.

To the secondary side of the transformer T3, a control unit 2 and the electrodes H1, H2, H5, H6 for supply electrode of the electrode belt 5, and the electrodes H3, H4, H7, H8 for detecting electrode are respectively connected.

Changing switches 47 and 48 are switched by switching control circuits 49 and 50 based on the CPU control, so as to select pulses as selected for the purpose, among the supplies to both hands; the neighborhood of the waist; hand- waists for the patterns as selected.

Further, switching between the impedance measurement and the treatment can be done by switching a switch 51 with a switching control circuit 52.

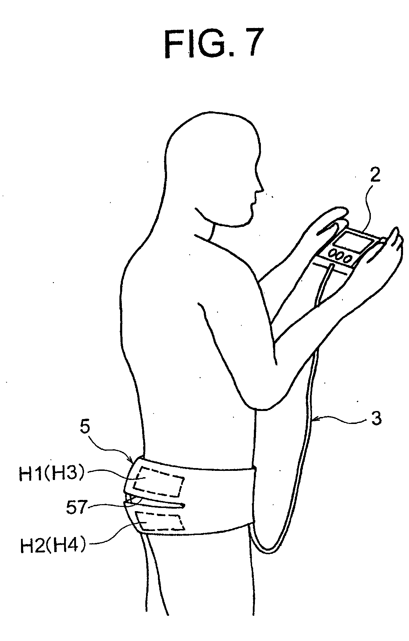

Referring to FIGS. 1 and 7, the structure of the electrode belt 5 will be described.

As shown in the drawings, the electrode belt 5 will be used on the neighborhood of the waist, buttocks and abdomen of the user by wrapping closely about the waist, buttocks and abdomen of the user with a face fastener 54. The electrode belt 5 may be made for example of a synthetic fibers made of urethane 18%, nylon 18% having high elasticity to form a base form 55 of the belt.

The planar electrodes H1, H2, H3 and H4 are formed on the contact surface of the base 55. Those planar electrodes H1, H2, H3 and H4 are formed by applying electrically conductive ink to the surface of the belt sheet 56 having insulation and flexibility, for example by gravure print and hot-pressing to melt and adhere on the surface. Further, the planar electrodes H1, H2, H3 and H4 can be formed by adhering an aluminium foil on the surface of the belt sheet 56 or by applying the mixture of metal powder and rubber or/and silicone powder.

Further, the electrode belt 5 has a slit 57 along the longer direction of the belt and between the upper electrodes H1 and H3 and the lower electrodes H2 and H4.

Accordingly, the electrode belt 5 can be installed along the waist of the user as shown in FIG. 7 as being broadened the belt the width of the belt with the slit. Therefore, the planar electrodes H1, H2, H3 and H4 can cover the wide range of the surface of the body, and then each portions of the body, such as buttocks, waist and abdomen can be treated at the same time.

The electrode belt 5 can be adjusted to any projections such as the buttocks, waist and abdomen of the user, to make close contact on the skin of the body of the user. Therefore, such simple structure of the electrode belt can make such projected surface of the body close contact, so that the planar electrodes H1, H2, H3 and H4 can be contacted stably on the body of the user, and then sufficient treatment effect can be expected.

Further, in the electrode belt 5 of this embodiment of the inventive health device, the direction to wrap the body (the longer direction of the belt) is normal or different (right angle) to the direction of the belt broadening by the slit (the shorter direction of the belt). Therefore, each position of the planar electrodes H1, H2, H3 and H4 contacting each the buttocks, waist and abdomen of the user may not be shifted during the treatment and keeping each position on the buttocks, waist and abdomen of the user, because the belt can be kept strongly wrapped by using the slit broadened.

Further, the slit 57 of the electrode belt 5 is not reaching the margin of the belt 5 (closed), that is, is formed merely in the middle of the belt 5. That means that the electrode belt 5 is separated at the middle thereof, and closed in both ends thereof, so that it can be easily handled when it is installed on the body of the user.

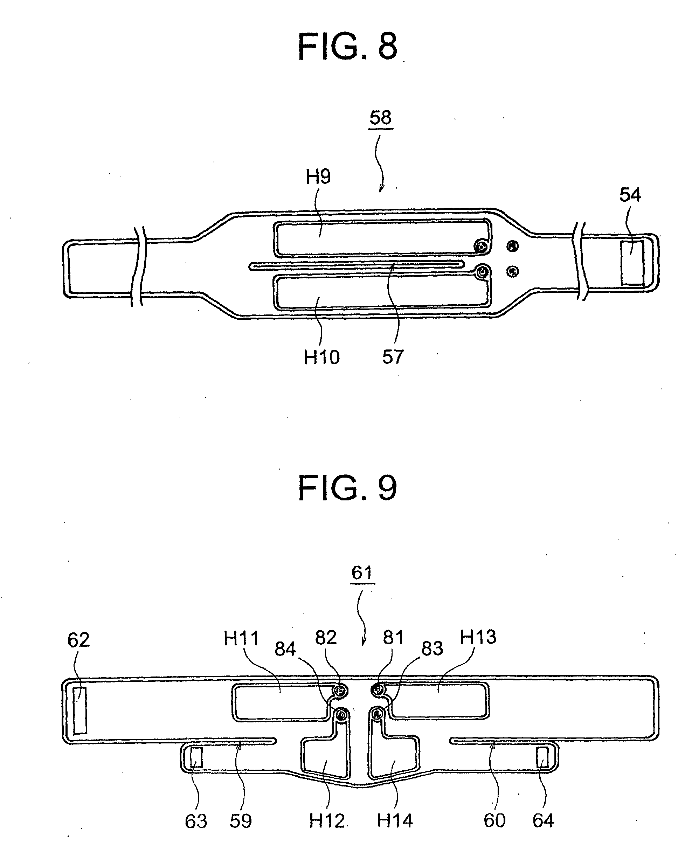

The pulse health device 1 of this embodiment has four planar electrodes, but alternatively the planar electrodes can be concentrated as shown in FIG. 8 into only two planar electrodes H9 and H10 of the belt 59. Then, the productivity of the belt can be improved.

As shown in FIG. 9, two slits 59 and 60 can be formed from the margin of the belt into the inside (middle) of the belt, open in the electrode belt 61, in accordance with one embodiment of the present invention. This figure of the belt will enhance the freedom of installation of the belt on the body of the user. The planar electrodes H11, H71 H13 and H14 each connecting the button projections 81, 82, 83 and 84 on which each the input pulse is charged are contacted under stable condition fixing each of the position of the electrodes for desired treatment.

Further, as shown in FIG. 9, face fastener s 62, 63, 64 can be provided in the belt so as to facilitate fixing of the belt on the body of the user. This is one alternative figure of the electrode belt.

### (Second Embodiment)

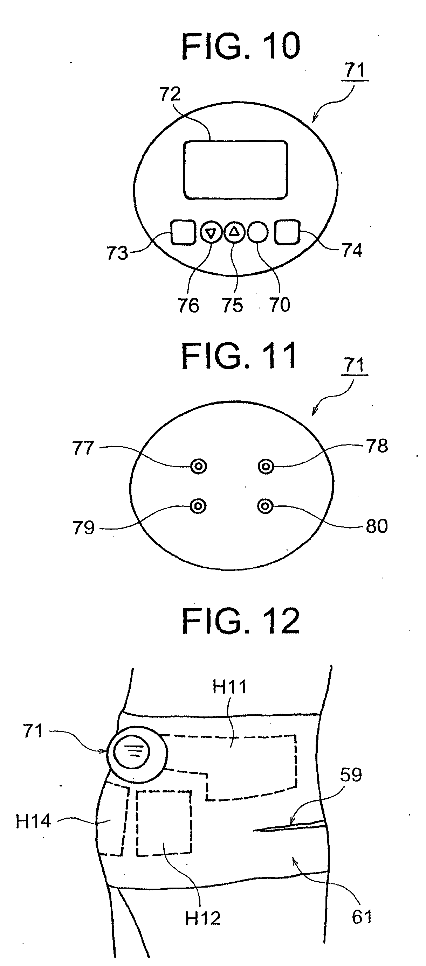

The second embodiment of the present invention will be described. FIG. 10 shows a front view of the control unit provided with the pulse health device, and FIG. 11 shows a back veiw of the control unit of FIG. 10, and FIG. 12 shows schematic view illustrating use of the control unit of FIG. 10 together with the conduct belt as installed on the weist of the user.

As shown in the drawings, this pulse health device of the second embodiment has a control unit 71 and the electrode belt 61 in place of the control unit 2, a cable 3 and an electrode belt 5 of the first embodiment.

The control unit 71 has a power source for generating pulse current (not shown in the drawings). There are provided in the front surface of the control unit 71, a display 72 for indicating visually a predetermined information for the user, a source, function selection button 73 for turning on or off, and selecting the functions, a start/stop button 74 for indicating the start and stop of the treatment, a up/down key 75 and 76 for setting the frequency of pulse current for the treatment, and setting the time of the treatment, and a set key 70. In the back of the control unit 71 (refer to FIG. 11), there are provided button recesses 77, 78, 79 and 80 for output connectors of the pulse current.

The button recesses 77, 78, 79 and 80 are as shown in FIG. 9 connect the planar electrodes H11, H12, H13 and H14 on the belt, which connect the button projections 81, 82, 83 and 84 for connecting the input.

The button projections 81, 82, 83 and 84 as provided concentratedly in the middle of the electrode belt 61 are detachablly installed respectively on the button recesses 77, 78, 79 and 80, and further, will hold the control unit 71 through the button recesses 77, 78, 79 and 80.

Accordingly, in the pulse health device of this embodiment, a cable to connect the control unit 71 and the electrode belt 61 is not necessary, so that the setting for starting the treatment can be easily carried out, and further, there is not any limitation during the treatment, and the user can move and exercise during the treatment.

### (Third Embodiment)

The pulse health device of this embodiment uses modifications of the above electrode belt 5, 58 and 61 as shown above.

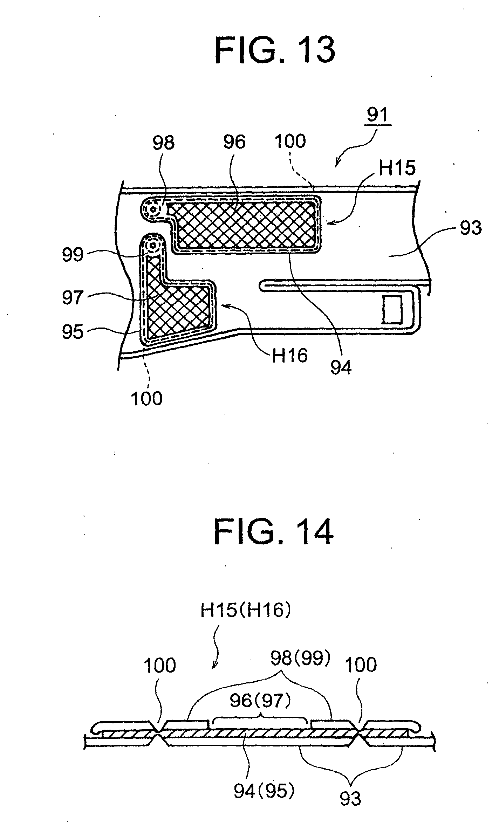

In reference to FIGS. 13 and 14 illustrating respectively a plane view and partly sectional view, the planar electrodes H15 and H16 of the electrode belt 91 are formed of electrically conductive sheets 94 and 95 formed on the surface of the belt 93 as made of urethane rubber and by coating the margin except of the electrode patterns 96 and 97 with electrically insulating coating 98 and 99.

The conduct sheet 94 and 95 can be formed by applying carbon ink on the surface of the belt 93 in a printing technique, or coating carbon material on the surface.

The electrically insulating coatings 98 and 99 are formed by applying insulating ink on the conductive sheets 94 and 95 by screen printing or gravure printing, or coating with insulating material, or painting with insulating paints, thermal melting of the insulating heat-meltable material, or adhering with insulating film.

The insulating ink and paint may be a mixture of a binder such as polyurethane resin or polyester resin and a solvent such as dimethylformaldehide and isophorone.

The heat-meltable material may be material which is solid under atmospheric temperature, and thermally elastic to be pourable or coatable easily when heated. Further, the heat-meltable material is used widely and practically for coating and adhering. In heat-melting method, heat and pressure may be applied on the insulating sheets made of this heat-meltable material to adhere them with the conductive sheets 94 and 95.

The electrically insulating coatings 98 and 99 may be formed by applying an insulating photosensitive material on the conductive sheets 94 and 95, and exposing through the electrode pattern and developing and fixing, or alternatively by adhering an insulating photosensitive thin film (dry film) on the surface of the conductive sheets 94 and 95, and exposing through the electrode pattern and developing and fixing.

The planar electrodes H15 and H16 may be sewed on the surface of the belt 93 together with the electrically insulating coatings 98 and 99 covering the conductive sheets 94 and 95. That is, the seam line 100 may be formed on the electrode belt 91. The button projections 83 and 84 connected to the planar electrodes H15 and H16 may be formed at the position where a part of the electrically insulating coatings 98 and 99 is removed to expose a part of the conductive sheets 94 and 95 from the insulating coatings 98 and 99.

Accordingly, the pulse health device of this embodiment can enable to enhance the contact-feel thereof by removing direct contacts to the skin of the user, by removing unevenness or projected margin of the seam line 100 of the planar electrodes H15 and H16, and further, removing direct contact of the edge of the planar electrodes H15 and H16.

The present invention is illustrated by a concrete embodiment, but is not limited to those embodiments, and can be modified without departing from the scope of the attached claims.

In this embodiment, the electrode belt 5 can be installed on the waist in order to treat the buttocks, waist and abdomen of the user. However, when one wishes to treat one's legs, the projections of the knees can be adjusted or absorbed by the slits of the belt so that the shins and thigh of the user can be simultaneously treated by modifying the structure of the belt 5. When one wishes to treat one's arms, the projections of the elbows can be adjusted or absorbed by the slits of the belt so that the above-elbows and below-elbows of the user can be simultaneously treated by modifying the structure of the belt 5.

Alternatively, pulse current can be applied under different conditions to different electrodes H1 and H3 and H2 and H4 each of which are positioned at the different sides of the slit 57. That is, when the planar electrodes H1 and H3 and the electrodes H2 and H4 are installed respectively on the wait (and abdomen) and the buttocks, the appropriate pulse currents can be applied or supplied under each different conditions, so that the effects of the treatment can be improved respectively.

### Industrial Utilization

As above described, the present invention is useful for manufacture of a device for shape-up treatment such as fitting up.

## Claims

1. A pulse health device; which comprises
a pulse generator for generating pulse current:
a belt provided with electrode(s) for providing the pulse current as generated by the pulse generator to a body of a user;
the belt having slits.

2. The health device as claimed in claim 1,
wherein the said slits are provided not reaching to the margin of the belt.

3. The health device as claimed in claim 1;
wherein said slits are formed from the margin of the belt toward the inside thereof.

4. The health device as claimed in claim 1;
wherein said slits are formed along the longer direction of the belt.

5. T he health device as claimed in claim 1;
wherein said slit is formed among at least two of the said electrodes as formed on the belt.

6. The health device as claimed in claim 1;
wherein the device is provided with said pulse generator and output connector or connectors for supply of pulse current;
said output connector(s) is mounted detachably on said belt, and
further input connector (s) mountablely provided on said belt, which can mount said device through said output connector.

7. The health device as claimed in claim 6;
wherein said belt is provided with a plurality of the electrodes which connects respectively said input connectors.

8. The health device as claimed in claim 1;
wherein said electrodes are formed as conductive sheets with the margin thereof is coated by insulating coatings.

9. The health device as claimed in claim 8;
wherein at least a part of said conductive sheet (s) is exposed from said insulating coatings to form input terminal(s) for said pulse current.

10. The health device as claimed in claim 8;
wherein said insulating coatings are formed as a coating of insulation on the surface of said conductive sheet(s).

11. The health device as claimed in claim 8;
wherein said insulating coatings are formed by coating insulating paint on the surface of said conductive sheet(s).

12. The health device as claimed in claim 8;
wherein said insulating coatings are formed by tharmal-melt coating of heat-meltably material having insulation property, on the surface of said conductive sheet(s).

13. The health device as claimed in claim 8;
wherein said insulating coatings are formed by adhering insulating film(s) on the surface of said conductive sheet(s).

14. The health device as claimed in claim 8;
wherein said insulating coatings are formed by coating insulating photo-sensitive material on the surface of said conductive sheet(s), and exposing through a pattern of electrode(s) to be formed, and developing and fixing.

15. The health device as claimed in claim 8;
wherein said insulating coatings are formed by adhering insulating photo-sensitive thin film on the surface of said conductive sheet(s), and exposing through a pattern of electrode(s) to be formed, and developing and fixing.
